# EUROPEAN PATENT APPLICATION

(11) **EP 4 434 532 A1**
(43) Date of publication of application: **25.09.2024**
(21) Application number: 24163531.7
(22) Date of filing: 14.03.2024
(51) Int. Cl.: A61K 36/28, A23L 33/105, A61K 8/00, A61K 31/77, A61K 36/734, A61K 36/78, A61K 36/87, A61P 13/02, A61P 15/10

(54) **PHOSPHODIESTERASE 5 INHIBITOR**

(30) Priority: 14.03.2023 JP 2023039865
(71) Applicant: ARKRAY, Inc., Kyoto-shi, Kyoto 601-8045 (JP)
(72) Inventor: Matsuo, Naoki, Kyoto-shi, Kyoto, 602-0008 (JP); Takayama, Kiyofumi, Koka-shi, Shiga, 520-3306 (JP); Yuasa, Eiji, Kyoto-shi, Kyoto, 602-0008 (JP); Takimura, Junichi, Kyoto-shi, Kyoto, 602-0008 (JP)
(74) Representative: Hoffmann Eitle

(57) **Abstract**

In one aspect, the present disclosure aims to provide a novel phosphodiesterase 5 inhibitor that uses plants as raw materials. One aspect of the present disclosure relates to a phosphodiesterase 5 inhibitor containing extracts of plants, wherein the plants are a plant of *Saururaceae Houttuynia,* a plant of *Rosaceae Crataegus,* a plant of *Compositae Chamaemelum,* and a plant of *Vitaceae Vitis.* Another aspect of the present disclosure relates to a food composition, beverage composition, health food, cosmetic, dietary supplement, quasi-drug, or pharmaceutical composition for preventing or ameliorating dysuria or ED, containing, as active ingredients, an extract(s) of a plant of *Saururaceae Houttuynia,* a plant of *Rosaceae Crataegus,* a plant of *Compositae Chamaemelum,* and a plant of *Vitaceae Vitis.*

## Description

### BACKGROUND OF THE INVENTION

### 1. Field of the Invention

The present disclosure relates to a phosphodiesterase 5 inhibitor, a food composition, beverage composition, health food, cosmetic, dietary supplement, quasi-drug, or pharmaceutical composition for inhibiting phosphodiesterase 5, and a method for producing a phosphodiesterase 5 inhibitor. The present disclosure also relates to a food composition, beverage composition, health food, cosmetic, dietary supplement, quasi-drug, or pharmaceutical composition for preventing or ameliorating erectile dysfunction (ED) or dysuria.

### 2. Description of Related Art

Phosphodiesterases (PDEs) are enzymes that hydrolyze phosphodiester bonds of cAMP, cGMP, etc., and play an important role in signal transduction. In mammals including humans, PDEs are classified into 11 families, which are characterized by their distinct expression sites and substrates. Attempts are currently made to improve or treat penile erectile dysfunction (ED), dysuria, etc. on the basis of, for example, smooth muscle relaxation induced by inhibiting PDE5 activity. There are various PDE5 inhibitory drugs such as, for example, sildenafil. Although therapeutic drugs containing a PDE5 inhibitory drug such as sildenafil as an active ingredient have been approved in Japan and many other countries, it is known that these drugs have side effects such as dizziness caused by a drop in blood pressure, headache, and hot flashes, for example. Besides, since these drugs are not over-the-counter drugs, anyone who wants to take these drugs must strictly follow the dosage regimen according to the prescription and guidance of a physician.

On the other hand, PDE5 inhibitors that use plants as raw materials also have been developed. Examples of such PDE5 inhibitors include a PDE5 activity inhibitor containing an extract extracted from *Astilbe thunbergii* (Siebold et Zucc.) Miq. (Japanese Patent No. 6981641), a PDE5 activity inhibitor containing an extract of a plant of the genus *Psidium* (JP 2019-034920A), and a PDE5A1 activity inhibitor containing at least one plant material selected from *Mangifera indica, Rhodiola rosea, Bacopa monnieri,* and *Hibiscus sabdariffa* (JP 2021-042152A). However, the effects of these inhibitors are not always sufficient, and there is demand for development of novel PDE5 inhibitors derived from plants.

The applicant of the present application has developed, for example, a Maillard reaction inhibitor (JP 2005-035911A), a flavor improving agent for foods and beverages (WO 2008/133284), a dehydroepiandrosterone production promoter (JP 2008-231031A), an oxidized protein hydrolase activity enhancer (WO 2011/004733), and a carboxymethylarginine production inhibitor and collagen denaturation inhibitor (WO 2011/004734), containing an extract of a plant of *Rosaceae Crataegus,* an extract of a plant *of Saururaceae Houttuynia,* an extract of a plant of *Vitaceae Vitis,* and/or an extract of a plant of *Compositae Chamaemelum.*

### SUMMARY OF THE INVENTION

The present disclosure provides a novel PDE5 inhibitor that uses plants as raw materials.

One aspect of the present disclosure relates to a phosphodiesterase 5 inhibitor containing extracts of plants, wherein the plants are a plant *of Saururaceae Houttuynia,* a plant of *Rosaceae Crataegus,* a plant of *Compositae Chamaemelum,* and a plant of *Vitaceae Vitis.*

Another aspect of the present disclosure relates to non-therapeutic use of a phosphodiesterase 5 inhibitor containing extracts of plants, wherein the plants are a plant of *Saururaceae Houttuynia,* a plant of *Rosaceae Crataegus,* a plant of *Compositae Chamaemelum,* and a plant of *Vitaceae Vitis.*

Still another aspect of the present disclosure relates to a food composition, beverage composition, health food, cosmetic, dietary supplement, quasi-drug, or pharmaceutical composition for inhibiting phosphodiesterase 5, containing the phosphodiesterase 5 inhibitor of the present disclosure.

Still another aspect of the present disclosure relates to a food composition, beverage composition, health food, cosmetic, dietary supplement, quasi-drug, or pharmaceutical composition for preventing or ameliorating dysuria or pulmonary arterial hypertension, containing, as active ingredients, an extract of a plant *of Saururaceae Houttuynia,* an extract of a plant of *Rosaceae Crataegus,* an extract of a plant of *Compositae Chamaemelum,* and an extract of a plant of *Vitaceae Vitis.*

Still another aspect of the present disclosure relates to a food composition, beverage composition, health food, cosmetic, dietary supplement, quasi-drug, or pharmaceutical composition for preventing or ameliorating ED, containing, as active ingredients, an extract of a plant of *Saururaceae Houttuynia,* an extract of a plant of *Rosaceae Crataegus,* an extract of a plant of *Compositae Chamaemelum,* and an extract of a plant of *Vitaceae Vitis.*

Still another aspect of the present disclosure relates to a method for producing a phosphodiesterase 5 inhibitor, including the step of: obtaining extracts of plants, wherein the plants are a plant *of Saururaceae Houttuynia,* a plant of *Rosaceae Crataegus,* a plant of *Compositae Chamaemelum,* and a plant of *Vitaceae Vitis,* and the step of obtaining the extracts includes subjecting the plants to hot water extraction.

Still another aspect of the present disclosure relates to a method for preventing or ameliorating a disease, including: supplying an effective amount of a composition containing extracts of plants as active ingredients to a subject in need thereof, wherein the plants are a plant of *Saururaceae Houttuynia,* a plant of *Rosaceae Crataegus,* a plant of *Compositae Chamaemelum,* and a plant of *Vitaceae Vitis,* and wherein the disease is selected from the group consisting of penile erectile dysfunction, dysuria, and pulmonary arterial hypertension.

The present disclosure can provide a novel PDE5 inhibitorthat uses a plant of *Saururaceae Houttuynia,* a plant of *Rosaceae Crataegus,* a plant of *Compositae Chamaemelum,* and a plant of *Vitaceae Vitis* as raw materials.

### BRIEF DESCRIPTION OF THE DRAWINGS

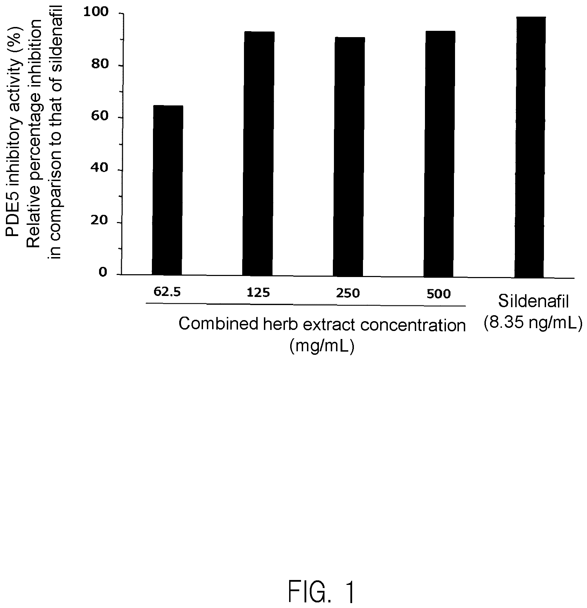

FIG. 1 is a graph showing the results of measuring PDE5 inhibitory activity in Example.

### DETAILED DESCRIPTION OF THE INVENTION

The present disclosure is based on the finding that a plant extract(s) obtained by extracting four types of plants, namely, plants of *Saururaceae Houttuynia, Rosaceae Crataegus, Compositae Chamaemelum,* and *Vitaceae Vitis,* can inhibit PDE5 activity.

### PDE5 Inhibitor

The PDE5 inhibitor of the present disclosure contains a plant extract(s) obtained by extracting four types of plants. More specifically, the PDE5 inhibitor of the present disclosure contains an extract(s) of plants of *Saururaceae Houttuynia, Rosaceae Crataegus, Compositae Chamaemelum,* and *Vitaceae Vitis.*

In one or more embodiments, examples of the plant *of Saururaceae Houttuynia* include *Houttuynia cordata.* In one or more embodiments, part of the plant to be subjected to extraction may be the whole plant or may be an aerial part(s) or underground part(s) of the plant, and preferably is an aerial part(s) of the plant. In one or more embodiments, examples of the aerial part include flowers, spikes, pericarps, fruits, stems, leaves, branches, branches and leaves, trunks, bark, and seeds. In one or more embodiments, examples of the underground part include rhizomes, root bark, and roots.

In one or more embodiments, examples of the plant of *Rosaceae Crataegus* include *Crataegus laevigata* and *Crataegus cuneata.* In one or more embodiments, part of the plant to be subjected to extraction may be the whole plant or may be one or more parts of the plant. In one or more embodiments, examples of the part of the plant to be subjected to extraction include flowers, spikes, pericarps, fruits, stems, leaves, branches, branches and leaves, trunks, bark, rhizomes, root bark, roots, and seeds, and, in particular, fruits are preferable.

In one or more embodiments, examples of the plant of *Compositae Chamaemelum* include *Chamaemelum nobile.* In one or more embodiments, part of the plant to be subjected to extraction may be the whole plant or may be one or more parts of the plant. In one or more embodiments, examples of the part of the plant to be subjected to extraction include flowers, spikes, flower stalks (inflorescence axes), pericarps, fruits, stems, leaves, rhizomes, root bark, roots, and seeds, and, in particular, anthodia (head inflorescences) are preferable. For *Chamaemelum nobile,* a scientific name such as *Ani-hemis nobilis* may also be used. Also, *Chamaemelum nobile* may be referred to as Roman chamomile.

Examples of the plant of *Vitaceae Vitis* include *Vitis vinifera, Vitis labrusca, Vitis saccharifera, Vitis ficifolia, Vii-is flexuosa, Vitis coiguetiae,* and *Vitis labruscana.* In one or more embodiments, part of the plant to be subjected to extraction may be the whole plant or may be one or more parts of the plant. In one or more embodiments, examples of the part of the plant to be subjected to extraction include flowers, spikes, pericarps, fruits, stems, leaves, branches, branches and leaves, trunks, bark, rhizomes, root bark, roots, and seeds, and, in particular, leaves are preferable.

The scientific names of plants in the present disclosure are based on the BG Plants Japanese-Scientific Names Index (Ylist) or the World Flora Online (WFO).

In one or more embodiments, the PDE5 inhibitor of the present disclosure contains an extract from an aerial part(s) of a plant *of Saururaceae Houttuynia,* an extract from fruits of a plant of *Rosaceae Crataegus,* an extract from anthodia of a plant of *Compositae Chamaemelum,* and an extract from leaves of a plant of *Vitaceae Vitis.* In one or more embodiments, the PDE5 inhibitor of the present disclosure contains an extract from a mixture containing an aerial part(s) of a plant of *Saururaceae Houttuynia,* fruits of a plant of *Rosaceae Crataegus,* anthodia of a plant of *Compositae Chamaemelum,* and leaves of a plant of *Vitaceae Vitis.*

In one or more embodiments, the PDE5 inhibitor of the present disclosure contains an extract of *Houttuynia cordata,* an extract of *Crataegus laevigata,* an extract of *Chamaemelum nobile,* and an extract of *Vitis vinifera.* In one or more embodiments, the PDE5 inhibitor of the present disclosure contains an extract of a mixture containing *Houttuynia cordata, Crataegus laevigata, Chamaemelum nobile,* and *Vitis vinifera.* In one or more embodiments, the PDE5 inhibitor of the present disclosure contains an extract from an aerial part(s) of *Houttuynia cordata,* an extract from fruits of *Crataegus laevigata,* an extract from anthodia of *Chamaemelum nobile,* and an extract from leaves of *Vitis vinifera,* and in other embodiments, the PDE5 inhibitor of the present disclosure contains an extract from at least one part of *Houttuynia cordata* selected from the group consisting of flowers, spikes, pericarps, stems, leaves, branches, branches and leaves, trunks, bark, and seeds, an extract from fruits of *Crataegus laevigata,* an extract from anthodia of *Chamaemelum nobile,* and an extract from leaves of *Vitis vinifera.*

The form of the extracts of the plants contained in the PDE5 inhibitor of the present disclosure can be determined as appropriate according to the form of the PDE5 inhibitor, and in one or more embodiments, the extracts of the plants may be in the form of liquid, paste, powder, or the like. In one or more embodiments, the PDE5 inhibitor of the present disclosure contains dried products of the extract(s) of the plants as active ingredients.

In one or more embodiments, the extract(s) of the plants contained in the PDE5 inhibitor of the present disclosure can be obtained by extracting any of the above-described parts and/or the whole plant body of the plants. In one or more embodiments, known methods can be used as a method for extracting, and examples thereof include solvent extraction and expression.

In one or more embodiments, the extracts of the plants in the PDE5 inhibitor of the present disclosure may be water extracts, organic solvent extracts, or the like. In one or more embodiments, the extracts of the plants in the PDE5 inhibitor of the present disclosure are hot water extracts obtained by subjecting the plants to hot water extraction.

In one or more embodiments, the extract(s) of the plants contained in the PDE5 inhibitor of the present disclosure may be prepared by: obtaining extracts from the respective plants and then mixing them together; mixing two or more of the four types of plants, subjecting the resultant mixture and the remaining plant(s) to extraction separately, and then mixing the obtained extracts together; or mixing all the four types of plants together and then subjecting the resulting mixture to extraction.

In one or more embodiments, the blending ratio (dry weight ratio) of an extract of a plant *of Saururaceae Houttuynia,* an extract of a plant of *Rosaceae Crataegus,* an extract of a plant of *Compositae Chamaemelum,* and an extract of a plant of *Vitaceae Vitis* in the PDE5 inhibitor of the present disclosure may be such that the amounts (weights) of the respective extracts are equal to or not equal to one another. In one or more embodiments, the blending ratio (dry weight ratio, A:B:C:D) of an extract (A) of a plant *of Saururaceae Houttuynia,* an extract (B) of a plant of *Rosaceae Crataegus,* an extract (C) of a plant of *Compositae Chamaemelum,* and an extract (D) of a plant of *Vitaceae Vitis* in the PDE5 inhibitor of the present disclosure may be 1:0.01-100:0.01-100:0.01-100, for example, and is preferably 1:0.1-10:0.1-10:0.1-10 and more preferably 1:0.5-5:0.5-5:0.5-5, for example. In one or more embodiments, the blending ratio (dry weight ratio, A1:B1:C1:D1) of an extract (A1) of *Houttuynia cordata,* an extract (B1) of *Crataegus laevigata,* an extract (C1) of *Chamaemelum nobile,* and an extract (D1) of *Vitis vinifera* in the PDE5 inhibitor of the present disclosure may be 1:0.01-100:0.01-100:0.01-100, for example, and is preferably 1:0.1-10:0.1-10:0.1-10 and more preferably 1:0.5-5:0.5-5:0.5-5, for example. In one or more embodiments, the blending ratio (dry weight ratio, A2:B2:C2:D2) of an extract (A2) from an aerial part(s) of *Houttuynia cordata,* an extract (B2) from fruits of *Crataegus laevigata,* an extract (C2) from anthodia of *Chamaemelum nobile,* and an extract (D2) from leaves of *Vitis vinifera* in the PDE5 inhibitor of the present disclosure may be 1:0.01-100:0.01-100:0.01-100, for example, and is preferably 1:0.1-10:0.1-10:0.1-10 and more preferably 1:0.5-5:0.5-5:0.5-5, for example.

In one or more embodiments, the PDE5 inhibitor of the present disclosure may consist of the extract(s) of the plants, or may further contain one or more freely selected other ingredients to the extent that the PDE5 inhibitory activity exhibited by the extracts is not impaired. In one or more embodiments, examples of the other ingredients include excipients, binders, lubricants, disintegrants, humectants, absorption promoters, emulsifying agents, stabilizers, preservatives, surfactants, diluents, solubilizing agents, antiseptic agents, taste masking agents, odor masking agents, colorants, and fragrances. In one or more embodiments, examples of the other ingredients include water, surfactants, glycerin, propylene glycol, alcohols, fats and oils, sugars, polysaccharides, gums, and polymer compounds. In one or more embodiments, examples of the alcohols include ethanol. In one or more embodiments, examples of the gums include gum arabic. In one or more embodiments, examples of the polymer compounds include dextrin. In one or more embodiments, the PDE5 inhibitor of the present disclosure may or may not further contain any ingredient having PDE5 inhibitory activity other than the extracts of the plants.

In one or more embodiments, the content of the extract(s) of the plants in the PDE5 inhibitor of the present disclosure is 0.1 wt% to 100 wt% in terms of dry weight. In one or more embodiments, the content in terms of dry weight is 1 wt% or more, 2 wt% or more, 5 wt% or more, 10 wt% or more, 20 wt% or more, 30 wt% or more, 40 wt% or more, 50 wt% or more, 60 wt% or more, or 70 wt% or more. Also, in one or more embodiments, the content in terms of dry weight is 99.9 wt% or less, 99 wt% or less, 98 wt% or less, 97 wt% or less, 96 wt% or less, 95 wt% or less, 90 wt% or less, 80 wt% or less, 75 wt% or less, or 70 wt% or less. In one or more embodiments, the content in terms of dry weight is 1 wt% to 99.9 wt%, 2 wt% to 99 wt%, 5 wt% to 98 wt%, 10 wt% to 97 wt%, 20 wt% to 96 wt%, 30 wt% to 95 wt%, 30 wt% to 90 wt%, 30 wt% to 80 wt%, 30 wt% to 75 wt%, or 30 wt% to 70 wt%.

In one or more embodiments, the PDE5 inhibitor of the present disclosure may be in the form of a tablet, granules, a capsule, or liquid, for example.

The intake of the PDE5 inhibitor of the present disclosure is not limited to a particular amount as long as desired effects are obtained, and may be set as appropriate according to, for example, the age, symptoms, and weight of a subject and the application form and intended use of the PDE5 inhibitor. In one or more embodiments, the daily intake of the PDE5 inhibitor of the present disclosure is 0.001 mg to 100 mg per 1 kg of body weight in terms of dry weight of the extracts of the plants contained in the PDE5 inhibitor. In one or more embodiments, the daily intake is 0.001 mg or more, 0.01 mg or more, 0.1 mg or more, or 1 mg or more, per 1 kg of body weight. In one or more embodiments, the daily intake is 100 mg or less, 50 mg or less, 20 mg or less, 10 mg or less, or 5 mg or less, per 1 kg of body weight. In one or more embodiments, the daily intake of the PDE5 inhibitor of the present disclosure may be a single intake or may be multiple intakes of two or more times.

In one or more embodiments, the PDE5 inhibitor of the present disclosure can inhibit PDE5 activity in vivo when it is taken and/or when it is administered to a living body. PDE5 is an enzyme that hydrolyzes phosphodiester bonds of cyclic guanosine monophosphate (cGMP), and is expressed in the corpus cavernosum, the lungs, the heart, blood vessels, and visceral muscles (visceral smooth muscles), as well as in platelets, etc. Inhibiting PDE5 activity can, for example, suppress a decrease in the cGMP concentration in smooth muscle cells and increase NO production. Accordingly, by inhibiting PDE5 activity, it is possible to, for example, relax muscles (smooth muscles) of the bladder, urethras, prostate, etc., thereby allowing vasodilatory action to be maintained and thus improving blood flow. Therefore, in one or more embodiments, the PDE5 inhibitor of the present disclosure can be used, for example, in preventing or ameliorating dysuria, ED (erectile dysfunction, decreased libido, erectile disorder, etc.), and pulmonary arterial hypertension, causing muscular relaxation of smooth muscles etc., and suppressing constriction of, e.g., blood vessels/the respiratory tract.

In one or more embodiments, the PDE5 inhibitor of the present disclosure can be used as a raw material of foods and beverages, such as food compositions, beverage compositions, and health foods, and as a raw material of cosmetics, dietary supplements, quasi-drugs, pharmaceutical compositions, and the like. Therefore, another aspect of the present disclosure relates to a raw material or food material (food ingredient) for imparting, to food compositions, beverage compositions, health foods, cosmetics, dietary supplements, quasi-drugs, or pharmaceutical compositions, a function of inhibiting PDE 5 and/or a function of, for example, preventing or ameliorating dysuria, ED, and/or pulmonary arterial hypertension, causing muscular relaxation, and suppressing constriction of, e.g., blood vessels/the respiratory tract. The raw material or food material of the present disclosure contains, as an active ingredient(s), the PDE5 inhibitor of the present disclosure; and/or an extract(s) of plants of *Saururaceae Houttuynia, Rosaceae Crataegus, Compositae Chamaemelum,* and *Vitaceae Vitis.*

Health foods generally refer to food products that claim to be good for health in general, and in one or more embodiments, examples of the health food include foods with health claims, which are foods that are labeled to state their functions in compliance with the standards etc. designated by a national government regarding their safety and effectiveness, such as foods for specified health uses, foods with nutrient function claims, and foods with functional claims. In one or more embodiments, the term "quasi-drug" as used in the present disclosure refers to a medicinal product, a nonmedicinal product, a quasi pharmaceutical composition, or a non-prescription drug. The quasi-drug may be a nonmedicinal product (a pharmaceutical composition) capable of exerting the effects and functions of the present disclosure.

The term "raw material of foods and beverages" as used in the present disclosure refers to a food ingredient or food material used in production of foods, including processed foods, nutritional supplementary foods, and health foods, and beverages. In one or more embodiments, the food ingredient or food material may be in the form of an aqueous solution, a concentrated solution, a dried product, or the like. In one or more embodiments, the form of the raw material is not limited to particular forms, and may be in the form of a dried product such as a solid, granules, or powder, paste, liquid, and the like.

In one or more embodiments, the PDE5 inhibitor of the present disclosure can be used as a food composition, beverage composition, health food, cosmetic, dietary supplement, quasi-drug, or pharmaceutical composition. Therefore, still another aspect of the present disclosure relates to a food composition, beverage composition, health food, cosmetic, dietary supplement, quasi-drug, or pharmaceutical composition, containing the PDE5 inhibitor of the present disclosure or containing the PDE5 inhibitor of the present disclosure as an active ingredient. Still another aspect of the present disclosure relates to a food composition, beverage composition, health food, cosmetic, dietary supplement, quasi-drug, or pharmaceutical composition, containing, as active ingredients, an extract(s) of a plant *of Saururaceae Houttuynia,* a plant of *Rosaceae Crataegus,* a plant of *Compositae Chamaemelum,* and a plant of *Vitaceae Vitis.* In one or more embodiments, the food composition, beverage composition, health food, cosmetic, dietary supplement, quasi-drug, or pharmaceutical composition of the present disclosure can be used for inhibiting PDE 5. In one or more embodiments, the food composition, beverage composition, health food, cosmetic, dietary supplement, quasi-drug, or pharmaceutical composition of the present disclosure can be used, for example, in preventing or ameliorating dysuria, ED, or pulmonary arterial hypertension, causing muscular relaxation, and suppressing constriction of, e.g., blood vessels/the respiratory tract.

In one or more embodiments, when the foods and beverages of the present disclosure, such as the food composition, beverage composition, and health food, are used in an adult human, they may be taken such that an approximate daily intake of the PDE5 inhibitor of the present disclosure or the extract(s) of the plants of *Saururaceae Houttuynia, Rosaceae Crataegus, Compositae Chamaemelum,* and *Vitaceae Vitis* is about 10 mg to about 1000 mg. In one or more embodiments, the approximate daily intake is 30 mg or more or 50 mg or more. In one or more embodiments, the approximate daily intake is 900 mg or less, 800 mg or less, or 700 mg or less. In one or more embodiments, the approximate daily intake is 30 mg to 900 mg, 30 mg to 800 mg, 30 mg to 700 mg, 50 mg to 800 mg, or 50 mg to 700 mg. In one or more embodiments, the foods and beverages of the present disclosure may be taken with meals or may be taken before, between, or after meals.

In one or more embodiments, the present disclosure may relate to use in animals including humans. In one or more embodiments, the use of the present disclosure may encompass therapeutic use and non-therapeutic use. The term "non-therapeutic use" as used in the present disclosure means use not involving surgery, therapy, or diagnosis practiced on humans. In one or more non-limiting embodiments, examples of the non-therapeutic use of the present disclosure include use in a subject who has not been diagnosed by a physician as having a disease such as penile erectile dysfunction, dysuria, or pulmonary arterial hypertension or having symptoms or conditions of such a disease but is in need of preventing or ameliorating the disease, symptoms, or conditions.

### Production Method of PDE5 Inhibitor

Still another aspect of the present disclosure relates to a method for producing a PDE5 inhibitor, including obtaining an extract(s) from a plant *of Saururaceae Houttuynia,* a plant of *Rosaceae Crataegus,* a plant of *Compositae Chamaemelum,* and a plant of *Vitaceae Vitis.* In one or more embodiments, the PDE5 inhibitor production method of the present disclosure can produce the PDE5 inhibitor of the present disclosure.

In one or more embodiments, the extracts can be obtained by extraction using particular parts of the plants or the whole plant bodies of the plants. In one or more embodiments, known methods can be used as a method for obtaining the extracts, and examples thereof include solvent extraction and expression. In one or more embodiments, the raw materials (plant bodies) used to obtain the extracts may be in a dry state or may be the plant bodies as they are (the plant bodies in a raw state before being dried).

In one or more embodiments, the raw materials used to obtain the extracts are preferably a dried product of an aerial part(s) of the plant *of Saururaceae Houttuynia,* a dried product of fruits of the plant of *Rosaceae Crataegus,* a dried product of anthodia of the plant of *Compositae Chamaemelum,* and a dried product of leaves of the plant of *Vitaceae Vitis.*

In one or more embodiments, an extraction solvent used in solvent extraction may be an aqueous solvent, an organic solvent, or the like. In one or more embodiments, examples of the aqueous solvent include water. In one or more embodiments, examples of the organic solvent include lower alcohols, polyhydric alcohols, ketones, esters, ethers, nitriles, aromatic compounds, and alkyl chlorides. In one or more embodiments, examples of the lower alcohols include methanol, ethanol, and dehydrated ethanol. In one or more embodiments, examples of the polyhydric alcohols include propylene glycol and 1,3-butylene glycol. In one or more embodiments, examples of the ketones include acetone and formic acid. In one or more embodiments, examples of the esters include ethyl acetate. In one or more embodiments, examples of the ethers include diethyl ether and dioxane. In one or more embodiments, examples of the nitriles include acetonitrile. In one or more embodiments, examples of the aromatic compounds include benzene, toluene, and xylene. In one or more embodiments, examples of the alkyl chlorides include chloroform. In one or more embodiments, one type of solvent may be used as the extraction solvent, or two or more types of solvents may be used in combination as extraction solvents. In one or more embodiments, the extraction solvent may be a mixed solvent containing an aqueous solvent and any of the above-described organic solvents. In one or more embodiments, the mixed solvent may be, for example, a lower alcohol aqueous solution such as an ethanol aqueous solution. In one or more embodiments, the proportion of the organic solvent in the mixed solvent is 1 vol% to 99 vol%.

In one or more embodiments, the PDE5 inhibitor production method of the present disclosure includes obtaining extracts of the plants by solvent extraction, and may optionally include mixing the extracts obtained separately from the respective plants together. In one or more embodiments, raw materials used to obtain the extracts may be subjected to treatments such as washing, drying, and pulverizing before being immersed.

In one or more embodiments, the extraction can be carried out by immersing parts to be subjected to extraction or the whole plant bodies (raw materials) of the plants in an extraction solvent. In one or more embodiments, the respective raw materials, namely, the plant of *Saururaceae Houttuynia,* the plant of *Rosaceae Crataegus,* the plant of *Compositae Chamaemelum,* and the plant of *Vitaceae Vitis,* may be subjected to extraction separately, they may be subjected to extraction after mixing two or more of the four types of plants, or they may be subjected to extraction after mixing all four types of plants (raw materials). In the case where the raw materials are subjected to extraction after mixing all four types of plants (raw materials), the ratio of the respective raw materials may be such that the amounts (weights) thereof are equal to or not equal to one another, in one or more embodiments. In one or more embodiments, the blending ratio (dry weight ratio, A':B':C':D') of a plant (A') *of Saururaceae Houttuynia,* a plant (B') of *Rosaceae Crataegus,* a plant (C') of *Compositae Chamaemelum,* and a plant (D') of *Vitaceae Vitis* may be 1:0.01-100:0.01-100:0.01-100, for example, and is preferably 1:0.1-10:0.1-10:0.1-10 and more preferably 1:0.5-5:0.5-5:0.5-5, for example.

In one or more embodiments, the production method of the present disclosure includes mixing the plant (A') *of Saururaceae Houttuynia,* the plant (B') of *Rosaceae Crataegus,* the plant (C') of *Compositae Chamaemelum,* and the plant (D') of *Vitaceae Vitis* at a blending ratio (dry weight ratio, A':B':C':D') of 1:0.01-100:0.01-100:0.01-100 and subjecting the thus-obtained mixture to hot water extraction. The blending ratio (dry weight ratio, A':B':C':D') is preferably 1:0.1-10:0.1-10:0.1-10 and more preferably 1:0.5-5:0.5-5:0.5-5, for example.

In one or more embodiments, the production method of the present disclosure includes mixing *Houttuynia cordata* (A1'), *Crataegus laevigata* (B1'), *Chamaemelum nobile* (C1'), and *Vitis vinifera* (D1') at a blending ratio (dry weight ratio, A1':B1':C1':D1') of 1:0.01-100:0.01-100:0.01-100 and subjecting the thus-obtained mixture to hot water extraction. The blending ratio (dry weight ratio, A1':B1':C1':D1') is preferably 1:0.1-10:0.1-10:0.1-10 and more preferably 1:0.5-5:0.5-5:0.5-5, for example.

In one or more embodiments, the ratio between a raw material to be subjected to extraction and an extraction solvent is 100 g (dry weight) of the raw material with respect to 1 Lto 1000 L of the extraction solvent and preferably with respect to 1 L to 100 L or 10 L to 80 L of the extraction solvent. The immersion time can be set as appropriate according to, for example, the type and the amount of the raw material and the type and the amount of the extraction solvent, and in one or more non-limiting embodiments, when 100 g (dry weight) of the raw material is immersed in 10 L of the extraction solvent, the immersion time is 0.5 hours or more, 1 hour or more, 1.5 hours or more, or 2 hours or more, or 24 hours or less, 22 hours or less, or 20 hours or less. The extraction (immersion) time is preferably 0.5 hours to 24 hours, 1 hour to 24 hours, 1.5 hours to 22 hours, or 2 hours to 20 hours.

In one or more embodiments, the temperature of the extraction solvent during extraction may be room temperature or may be equal to or higherthan room temperature or equal to or lower than room temperature. When the extraction solvent is an aqueous solvent, the extraction is preferably hot water extraction in one or more embodiments. The temperature of the aqueous solvent in the case of hot water extraction is 30°C or more, 40°C or more, 50°C or more, 55°C or more, 60°C or more, 65°C or more, 70°C or more, or 75°C or more, or 100°C or less, 95°C or less, or 90°C or less, in one or more embodiments. The temperature of the aqueous solvent in the case of hot water extraction is preferably 50°C to 100°C, 60°C to 100°C, 60°C to 95°C, 65°C to 90°C, or 70°C to 90°C. The treatment time in hot water extraction can be set as appropriate according to, for example, the type and the amount of the raw material and the amount of the aqueous solvent, and in one or more non-limiting embodiments, when 100 g (dry weight) of the raw material is immersed in 10 L of the aqueous solvent, the extraction (immersion) time is 0.5 hours or more, 1 hour or more, 1.5 hours or more, or 2 hours or more, or 24 hours or less, 22 hours or less, or 20 hours or less. The extraction (immersion) time is preferably 0.5 hours to 24 hours, 1 hour to 24 hours, or 2 to 20 hours.

After the extraction, the obtained extract may be subjected to a further treatment such as purification. The purification may be performed using a known method, and in one or more embodiments, examples thereof include distillation, filtration, chromatography, and drying.

### Method for Producing Raw Materials and Food Materials

Still another aspect of the present disclosure relates to a method for producing a raw material or food material (food ingredient) for imparting, to food compositions, beverage compositions, health foods, cosmetics, dietary supplements, quasi-drugs, or pharmaceutical compositions, a function of inhibiting PDE 5 and/or a function of, for example, preventing or ameliorating dysuria, ED, and/or pulmonary arterial hypertension, causing muscular relaxation, and suppressing constriction of, e.g., blood vessels/the respiratory tract, including obtaining an extract(s) from a plant *of Saururaceae Houttuynia,* a plant of *Rosaceae Crataegus,* a plant of *Compositae Chamaemelum,* and a plant of *Vitaceae Vitis.* The step of obtaining the extracts in the production method of this aspect can be performed in the same manner as in the PDE5 inhibitor production method of the present disclosure.

### Method for Producing Beverage Composition, Food Composition, etc.

Still another aspect of the present disclosure relates to a method for producing the PDE5 inhibitor of the present disclosure and/or a food composition, beverage composition, health food, cosmetic, dietary supplement, or quasi-drug, containing an extract(s) of a plant *of Saururaceae Houttuynia,* a plant of *Rosaceae Crataegus,* a plant of *Compositae Chamaemelum,* and a plant of *Vitaceae Vitis.* In one or more embodiments, the production method includes: adding the PDE5 inhibitor of the present disclosure and/or the extract(s) of the plants of *Saururaceae Houttuynia, Rosaceae Crataegus, Compositae Chamaemelum,* and *Vitaceae Vitis* to a food composition, beverage composition, health food, cosmetic, dietary supplement, quasi-drug, or pharmaceutical composition of interest; and/or mixing the same with raw materials of the food composition, beverage composition, health food, cosmetic, dietary supplement, quasi-drug, or pharmaceutical composition of interest.

### Method for Preventing or Ameliorating Diseases

Still another aspect of the present disclosure relates to a method for preventing or ameliorating diseases such as penile erectile dysfunction, dysuria, and pulmonary arterial hypertension on the basis of smooth muscle relaxation. The prevention or amelioration method of the present disclosure includes supplying an effective amount of a composition containing extracts of plants as active ingredients to a subject in need thereof, and the plants are a plant of *Saururaceae Houttuynia,* a plant of *Rosaceae Crataegus,* a plant of *Compositae Chamaemelum,* and a plant of *Vitaceae Vitis.*

In one or more embodiments, the prevention or amelioration method of the present disclosure includes supplying the composition to the subject in need thereof once or at least once. There is no particular limitation on the number of times the composition is supplied, intervals at which the composition is supplied, a period for which the composition is supplied, etc., and they can be determined as appropriate according to the age, weight, and conditions of the subject. In one or more embodiments, the number of times the composition is supplied per day may be once or two or more times.

In one or more embodiments, the composition containing the extracts of the plants as active ingredients may be the PDE 5 inhibitor, food composition, beverage composition, health food, cosmetic, dietary supplement, quasi-drug, or pharmaceutical composition according to the present disclosure.

The present disclosure can relate to the following one or more non-limiting embodiments.
[A1] A phosphodiesterase 5 inhibitor containing:
   an extract(s) of plants,
   wherein the plants are a plant *of Saururaceae Houttuynia,* a plant of *Rosaceae Crataegus,* a plant of *Compositae Chamaemelum,* and a plant of *Vitaceae Vitis.*
[A2] The phosphodiesterase 5 inhibitor according to [A1],
   wherein the plant *of Saururaceae Houttuynia* is *Houttuynia cordata,* the plant of *Rosaceae Crataegus* is *Crataegus laevigata,* the plant of *Compositae Chamaemelum* is *Chamaemelum nobile,* and the plant of *Vitaceae Vitis* is *Vitis vinifera.*
[A3] The phosphodiesterase 5 inhibitor according to [A1] or [A2],
   wherein the extracts are at least one of water extracts or organic solvent extracts.
[A4] The phosphodiesterase 5 inhibitor according to any one of [A1] to [A3],
   wherein dried products of the extracts are contained as active ingredients.
[A5] A food composition, beverage composition, health food, cosmetic, dietary supplement, quasi-drug, or pharmaceutical composition for inhibiting phosphodiesterase 5, containing:
   the phosphodiesterase 5 inhibitor according to any one of [A1] to [A4].
[A6] A food composition, beverage composition, health food, cosmetic, dietary supplement, quasi-drug, or pharmaceutical composition for preventing or ameliorating dysuria or pulmonary arterial hypertension, containing, as active ingredients:
   an extract(s) of a plant *of Saururaceae Houttuynia,* a plant of *Rosaceae Crataegus,* a plant of *Compositae Chamaemelum,* and a plant of *Vitaceae Vitis.*
[A7] A food composition, beverage composition, health food, cosmetic, dietary supplement, quasi-drug, or pharmaceutical composition for preventing or ameliorating ED, containing, as active ingredients:
   an extract(s) of a plant *of Saururaceae Houttuynia,* a plant of *Rosaceae Crataegus,* a plant of *Compositae Chamaemelum,* and a plant of *Vitaceae Vitis.*
[A8] A method for producing a phosphodiesterase 5 inhibitor, including the step of:
   obtaining extracts of plants,
   wherein the plants are a plant *of Saururaceae Houttuynia,* a plant of *Rosaceae Crataegus,* a plant of *Compositae Chamaemelum,* and a plant of *Vitaceae Vitis,* and
   the step of obtaining the extracts includes subjecting the plants to hot water extraction.
[A9] The method according to [A8], further includes drying the extracts obtained by the hot water extraction.
[A10] The method according to [A8] or [A9],
   wherein the step of obtaining the extracts includes subjecting a dried product of an aerial part of the plant *of Saururaceae Houttuynia,* a dried product of a fruit of the plant of *Rosaceae Crataegus,* a dried product of an anthodium of the plant of *Compositae Chamaemelum,* and a dried product of a leaf of the plant of *Vitaceae Vitis* to the hot water extraction.
[A11] The method according to any one of [A8] to [A10],
   wherein the step of obtaining the extracts includes separately subjecting the plant of *Saururaceae Houttuynia,* the plant of *Rosaceae Crataegus,* the plant of *Compositae Chamaemelum,* and the plant of *Vitaceae Vitis* to the hot water extraction and mixing the thus-obtained extracts of the plant *of Saururaceae Houttuynia,* the plant of *Rosaceae Crataegus,* the plant of *Compositae Chamaemelum,* and the plant of *Vitaceae Vitis* together.
[A12] The method according to any one of [A8] to [A10],
   wherein the step of obtaining the extracts includes mixing two, three, or four types of plants selected from the group consisting of the plant *of Saururaceae Houttuynia,* the plant of *Rosaceae Crataegus,* the plant of *Compositae Chamaemelum,* and the plant of *Vitaceae Vitis* together and subjecting the thus-obtained mixture to the hot water extraction.
[A13] The method according to [A12],
   wherein the mixing includes mixing two, three, or four types of dried products selected from the group consisting of a dried product of an aerial part of the plant *of Saururaceae Houttuynia,* a dried product of a fruit of the plant of *Rosaceae Crataegus,* a dried product of an anthodium of the plant of *Compositae Chamaemelum,* and a dried product of a leaf of the plant of *Vitaceae Vitis* together.
[A14] The method according to any one of [A8] to [A13],
   wherein the phosphodiesterase 5 inhibitor is the phosphodiesterase 5 inhibitor according to any one of [A1] to [A4].
[A15] Use of an extract of a plant *of Saururaceae Houttuynia,* an extract of a plant of *Rosaceae Crataegus,* an extract of a plant of *Compositae Chamaemelum,* and an extract of a plant of *Vitaceae Vitis* in production of a pharmaceutical composition for preventing or ameliorating dysuria, ED, or pulmonary arterial hypertension.
[B1] Non-therapeutic use of a phosphodiesterase 5 inhibitor containing an extract(s) of plants,
   wherein the plants are a plant *of Saururaceae Houttuynia,* a plant of *Rosaceae Crataegus,* a plant of *Compositae Chamaemelum,* and a plant of *Vitaceae Vitis.*
[B2] The non-therapeutic use according to [B1],
   wherein the plant *of Saururaceae Houttuynia* is *Houttuynia cordata,* the plant of *Rosaceae Crataegus* is *Crataegus laevigata,* the plant of *Compositae Chamaemelum* is *Chamaemelum nobile,* and the plant of *Vitaceae Vitis* is *Vitis vinifera.*
[B3] The non-therapeutic use according to claim [B1] or [B2],
   wherein the extracts are at least one of water extracts or organic solvent extracts.
[B4] The non-therapeutic use according to any one of claims [B1] to [B3],
   wherein the phosphodiesterase 5 inhibitor contains dried products of the extracts as active ingredients.
[B5] The non-therapeutic use according to any of claims [B1] to [B4],
   wherein the phosphodiesterase 5 inhibitor further contains at least one selected from the group consisting of surfactants, glycerin, propylene glycol, alcohols, fats and oils, sugars, polysaccharides, gums, and polymer compounds.
[B6] The non-therapeutic use according to any one of claims [B1] to [B5],
   wherein a content of the extracts of the plants in the phosphodiesterase 5 inhibitor is 30 wt% to 80 wt% in terms of dry weight.
[B7] The non-therapeutic use according to any one of claims [B1] to [B6],
   wherein the extracts of the plants are contained as active ingredients in a food composition, a beverage composition, a health food, a cosmetic, a dietary supplement, a quasi-drug, or a pharmaceutical composition.
[C1] A pharmaceutical composition for use in prevention or amelioration of at least one selected from dysuria and penile erectile dysfunction, the pharmaceutical composition comprising, as active ingredients, an extract(s) of a plant *of Saururaceae Houttuynia,* a plant of *Rosaceae Crataegus,* a plant of *Compositae Chamaemelum,* and a plant of *Vitaceae Vitis.*
[D1] A method for preventing or ameliorating dysuria, the method comprising:
   supplying an effective amount of a composition containing extracts of plants as active ingredients to a subject in need thereof,
   wherein the plants are a plant *of Saururaceae Houttuynia,* a plant of *Rosaceae Crataegus,* a plant of *Compositae Chamaemelum,* and a plant of *Vitaceae Vitis.*
[E1] A method of preventing or ameliorating penile erectile dysfunction, the method comprising:
   supplying an effective amount of a composition containing extracts of plants as active ingredients to a subject in need thereof,
wherein the plants are a plant *of Saururaceae Houttuynia,* a plant of *Rosaceae Crataegus,* a plant of *Compositae Chamaemelum,* and a plant of *Vitaceae Vitis.*
[F1] A method for inhibiting phosphodiesterase 5 activity in a subject, the method comprising:
   supplying (administrating) an effective amount of a composition containing extracts of plants as active ingredients to a subject in need thereof,
   wherein the plants are a plant *of Saururaceae Houttuynia,* a plant of *Rosaceae Crataegus,* a plant of *Compositae Chamaemelum,* and a plant of *Vitaceae Vitis.*
[G1] A method for inhibiting phosphodiesterase 5 activity in a sample, the method comprising:
   adding a composition containing extracts of plants as active ingredients to a sample,
   wherein the plants are a plant *of Saururaceae Houttuynia,* a plant of *Rosaceae Crataegus,* a plant of *Compositae Chamaemelum,* and a plant of *Vitaceae Vitis.*

Hereinafter, the present disclosure will be described in further detail by way of an example. However, this example is merely illustrative, and the present disclosure is not limited thereto.

### EXAMPLE

### Example of Preparation of Plant Extracts

An aerial part of *Houttuynia cordata,* fruits of *Crataegus laevigata,* anthodia of *Chamaemelum nobile,* and leaves of *Vitis vinifera* were dried and pulverized, and the thus-obtained four types of herbs were mixed together. The dry mixture (100 g) was immersed in purified water (10 L) at about 80°C for about 5 hours. Then, the residue was removed by filtering, and the filtrate (about 10 kg) was collected. The collected filtrate was dried to remove the solvent (purified water), whereby an extract (20 g) in the form of powder was obtained. Thus, the obtained extract was mixed with dextrin, and the resultant mixture was dried. Thus, a powdery herb extract containing the above-described four types of herbs was prepared. The extract and the dextrin were mixed in such a manner that the ratio of the dextrin to the solid content in the extract (concentrate) was 4:6 (extract:dextrin).

### Measurement of PDE5 Inhibitory Activity

PDE5 inhibitory activity was measured according to the protocol of a PDE-Glo Phosphodiesterase assay kit (Promega) using PDE5A, GST-Tag (BPS Bioscience) as an enzyme. The combined herb extract was used in measurement of PDE5 inhibitory activity, and the PDE5 inhibitory activity of the combined herb extract at different concentrations was evaluated.

The final concentrations of the combined herb extract were set to 62.5 µg/mL, 125 µg/mL, 250 µg/mL, and 500 µg/mL, and the PDE inhibitory activity was evaluated at each of these concentrations. Also, sildenafil, which is a PDE5 inhibitory drug, was used as a positive control test compound.

### Measurement Procedure

1. Samples (combined herb extracts) were each prepared as appropriate so as to have a concentration twice as high as the desired final concentration using PDE-Glo reaction buffer (5×) included in the PDE-Glo Phosphodiesterase assay kit.
2.25 µL of each sample was added to a 96-well plate.
3.12.5 µL of a PDE5A solution was added to each of the wells containing the sample, and the plate was agitated for 1 minute on a plate mixer. After the agitation, the wells were incubated at 25°C for 4 minutes.
4.12.5 µL of 2.5 µM cGMP solution was added as a substrate to each of the incubated wells, and the plate was agitated for 1 minute on the plate mixer. After the agitation, the wells were incubated at 25°C for 90 minutes.
5.12.5 µL of PDE-Glo termination buffer included in the PDE-Glo Phosphodiesterase assay kit was added to each of the incubated wells, and the plate was agitated for 1 minute using a plate mixer. After the agitation, the wells were incubated at 25°C for 20 minutes.
6.12.5 µL of detection solution included in the PDE-Glo Phosphodiesterase assay kit was added to each of the incubated wells, and the plate was agitated for 1 minute on the plate mixer. After the agitation, the wells were incubated at 25°C for 20 minutes.
7.50 µL of Kinase-Glo reagent included in the PDE-Glo Phosphodiesterase assay kit was added to each of the incubated wells, and the plate was agitated for 1 minute using a plate mixer. After the agitation, the wells were incubated at 25°C for 10 minutes.
8. Luminescence (RLU) measurement was performed using a fluorescence plate reader (trade name: MTP-700CL, CORONA ELECTRIC Co., Ltd.).
9. The same measurement was performed using sildenafil (8.35 ng/mL) as a positive control.
10. The relative percentage inhibition achieved by the sample was determined with the percentage inhibition achieved by the positive control, sildenafil (8.35 ng/mL), taken as 100%. An example of the results obtained is shown in Table 1 below and FIG. 1.

**[Table 1]**

| PDE5 Inhibitory Activity (the relative percentage inhibition with the percentage inhibition achieved by 8.35 ng/mL sildenafil taken as 100%) | | | | | |
|---|---|---|---|---|---|
| Sample Name and Concentration | Mixed Herbal Extract (µg/mL) | | | | Sildenafil (ng/mL) |
| | 62.5 | 125 | 250 | 500 | 8.35 |
| PDE5 inhibitory Activity (%) | 64.7 | 93.2 | 91.4 | 94.2 | 100 |

As can be seen from Table 1 and FIG. 1, the samples at all the concentrations exhibited the PDE5 inhibitory activity comparable to that of the positive control, sildenafil. In particular, the samples with a final concentration of 125, 250, and 500 µg/mL exhibited the PDE5 inhibitory activity at approximately the same level as that of the positive control, sildenafil.

Thus, the results suggest that a composition containing extracts of four types of herbs, namely, *Houttuynia cordata, Crataegus laevigata, Chamaemelum nobile,* and *Vitis vinifera,* is effective in, for example, preventing or ameliorating dysuria, ED, etc. and in muscle relaxation or suppression of constriction of blood vessels/the respiratory tract.

The invention may be embodied in other forms without departing from the spirit or essential characteristics thereof. The embodiments disclosed in this application are to be considered in all respects as illustrative and not limiting. The scope of the invention is indicated by the appended claims rather than by the foregoing description, and all changes that come within the meaning and range of equivalency of the claims are intended to be embraced therein.

## Claims

1. A phosphodiesterase 5 inhibitor containing an extract(s) of plants,
wherein the plants are a plant of Saururaceae Houttuynia, a plant of Rosaceae Crataegus, a plant of Compositae Chamaemelum, and a plant of Vitaceae Vitis.

2. The phosphodiesterase 5 inhibitor according to claim 1,
wherein the plant of Saururaceae Houttuynia is Houttuynia cordata, the plant of Rosaceae Crataegus is Crataegus laevigata, the plant of Compositae Chamaemelum is Chamaemelum nobile, and the plant of Vitaceae Vitis is Vitis vinifera.

3. The phosphodiesterase 5 inhibitor according to claim 1 or 2,
wherein the extracts are at least one of water extracts or organic solvent extracts; and/or the phosphodiesterase 5 inhibitor contains dried products of the extracts as active ingredients.

4. The phosphodiesterase 5 inhibitor according to any of claims 1 to 3,
wherein the phosphodiesterase 5 inhibitor further contains at least one selected from the group consisting of surfactants, glycerin, propylene glycol, alcohols, fats and oils, sugars, polysaccharides, gums, and polymer compounds; and/or a content of the extracts of the plants in the phosphodiesterase 5 inhibitor is 30 wt% to 80 wt% in terms of dry weight

5. A food composition, a beverage composition, a health food, a cosmetic, a dietary supplement, a quasi-drug, or a pharmaceutical composition containing the phosphodiesterase 5 inhibitor according to any one of claims 1 to 4 as active ingredients.

6. A method for producing the phosphodiesterase 5 inhibitor according to any one of claims 1 to 4, the method comprising the step of:
obtaining extracts of plants,
wherein the plants are a plant of Saururaceae Houttuynia, a plant of Rosaceae Crataegus, a plant of Compositae Chamaemelum, and a plant of Vitaceae Vitis, and
the step of obtaining the extracts includes subjecting the plants to hot water extraction.

7. The method according to claim 6,
wherein the step of obtaining the extracts includes subjecting a dried product of an aerial part of the plant of Saururaceae Houttuynia, a dried product of a fruit of the plant of Rosaceae Crataegus, a dried product of an anthodium of the plant of Compositae Chamaemelum, and a dried product of a leaf of the plant of Vitaceae Vitis to the hot water extraction.

8. The method according to claim 6 or 7, further comprising drying the extracts obtained by the hot water extraction.

9. The method according to any one of claims 6 to 8,
wherein the plants subjected to the hot water extraction are dried products of the plants; and/or
wherein the hot water extraction includes obtaining the extracts of the plants after mixing two or more types of plants selected from the group consisting of the plant of Saururaceae Houttuynia, the plant of Rosaceae Crataegus, the plant of Compositae Chamaemelum, and the plant of Vitaceae Vitis.

10. The method according to any one of claims 6 to 9,
wherein the hot water extraction includes immersing the plants in an aqueous solvent at 60°C to 100°C.

11. The method according to any one of claims 6 to 10,
wherein a blending ratio of raw materials used in the hot water extraction is as follows: a blending ratio (dry weight ratio, A':B':C':D') of the plant (A') of Saururaceae Houttuynia, the plant (B') of Rosaceae Crataegus, the plant (C') of Compositae Chamaemelum, and the plant (D') of Vitaceae Vitis is 1:0.01-100:0.01-100:0.01-100.

12. The method according to any of claims 6 to 11, further comprising mixing the obtained extracts of the plants with at least one selected from the group consisting of surfactants, glycerin, propylene glycol, alcohols, fats and oils, sugars, polysaccharides, gums, and polymer compounds.

13. The phosphodiesterase 5 inhibitor according to any one of claims 1 to 4 or the food composition, the beverage composition, the health food, the cosmetic, the dietary supplement, the quasi-drug, or the pharmaceutical composition according to claim 5 for non-therapeutic use.

14. The phosphodiesterase 5 inhibitor according to any one of claims 1 to 4 or the food composition, the beverage composition, the health food, the cosmetic, the dietary supplement, the quasi-drug, or the pharmaceutical composition according to claim 5 for use in a method of prevention or amelioration a disease, wherein the disease is preferably selected from dysuria and penile erectile dysfunction.

15. The phosphodiesterase 5 inhibitor, the food composition, the beverage composition, the health food, the cosmetic, the dietary supplement, the quasi-drug, or the pharmaceutical composition for use according to claim 14, wherein the method comprises:
supplying an effective amount of the phosphodiesterase 5 inhibitor, the food composition, the beverage composition, the health food, the cosmetic, the dietary supplement, the quasi-drug, or the pharmaceutical composition to a subject in need thereof.
